# EUROPEAN PATENT SPECIFICATION

(11) **EP 1 077 260 B1**
(45) Date of publication and mention of the grant of the patent: **31.03.2010**
(21) Application number: 99115161.4
(22) Date of filing: 13.08.1999
(51) Int. Cl.: C12N 15/35, C07K 14/015, C07K 16/08, G01N 33/569, C12Q 1/70, A61K 35/76, A61K 48/00

(54) **Parvovirus NS1 variants**
Parvovirus NS1 Varianten
Variants du Parvovirus NS1

(43) Date of publication of application: 21.02.2001
(73) Proprietor: Deutsches Krebsforschungszentrum Stiftung des öffentlichen Rechts, 69120 Heidelberg (DE)
(72) Inventor: Nüesch, Jürg, Dr., 4132 Muttenz (CH); Rommelaere, Jean, Prof. Dr., 69118 Heidelberg (DE)
(74) Representative: Schüssler, Andrea

(56) References cited:
- D LEGENDRE & J ROMMELAERE: "Terminal regions of the NS-1 protein of the parvovirus Minute Virus of Mice are involved in cytotoxicity and promoter trans inhibition" JOURNAL OF VIROLOGY, vol. 66, no. 10, October 1992 (1992-10), pages 5705-5713, XP000867510 AMERICAN SOCIETY FOR MICROBIOLOGY US
- LI X ET AL: "Mutation of lysine 405 to serine in the parvovirus H-1 NS1 abolishes its function for viral DNA replication, late promoter activation, and cytotoxocity" JOURNAL OF VIROLOGY, vol. 64, no. 10, October 1990 (1990-10), pages 4654-4660, XP000867496 AMERICAN SOCIETY FOR MICROBIOLOGY US
- J P F NÜESCH ET AL: "Sequence motifs in the replicator protein of parvovirus MVM essential for nicking and covalent attachement to the viral origin: identification of the linking tyrosine" VIROLOGY,US,ACADEMIC PRESS,ORLANDO, vol. 209, no. 1, 10 May 1995 (1995-05-10), pages 122-135, XP002088311 ISSN: 0042-6822
- S F COTTMORE ET AL: "The NS1 polypeptide of the murine parvovirus MVM binds to DNA sequences containing the motif (ACCA)2-3" JOURNAL OF VIROLOGY,US,THE AMERICAN SOCIETY FOR MICROBIOLOGY, vol. 69, no. 3, pages 1652-1660, XP002088309 ISSN: 0022-538X
- JPF NÜESCH ET AL.: 'Biochemical activities of minute virus of mice nonstructural protein NS1 are modulated in vitro by the phosphorylation state of the polypeptide' JOURNAL OF VIROLOGY vol. 72, no. 10, October 1998, pages 8002 - 8012

## Description

The present invention relates to the use of parvovirus NS1 variants as further characterized in the claims. Thus, it relates to the use of a parvovirus NS 1 variant having a shifted equilibrium between the DNA replication and transcription activities (a) and the cytotoxicity activity (b), wherein one or several phosphorylation sites are mutated by amino acid substitution causing the shift of equilibrium; and
(i) the activities (a) are reduced or eliminated, and activity (b) is maintained or increased or
(ii) the activities (a) are maintained and activity (b) is reduced or eliminated for the preparation of a pharmaceutical composition for treating tumoral diseases.

Parvovirus designates a genus of the virus family Parvoviridae. The parvovirus genus comprises a number of small, icosaedric viruses that can replicate in the absence of a helper virus. Parvovirus contains a single-stranded DNA having a length of about 5.000 bp. At the 3' and 5' ends of the DNA there is one palindromic sequence each. The DNA codes for two capsid proteins, VP1 and VP2, as well as for two regulatory non-structure proteins, NS-1 and NS-2. The latter proteins are phosphorylated and show nuclear or both cytoplasmic and nuclear localization, respectively. NS1 is necessary for viral DNA replication and participates in the regulation of viral gene expression. Particularly, NS1 transactivates the promoter P38 and exhibits DNA-binding, helicase and DNA-nicking activities. Furthermore, NS1 induces cytotoxic and/or cytostatic stress in sensitive host cells.

Parvoviruses are usually well-tolerated by populations of their natural host, in which they persist without apparent pathological signs. This is due to both the protection of foetuses and neonates by maternal immunity, and the striking restriction of parvovirus replication to a narrow range of target proliferating tissues in adult animals. This host tolerance concerns especially rodent parvoviruses, for example the minute virus of mice (MVM) and H-1 virus in their respective natural hosts, namely mice and rats. In addition, humans can be infected with the latter viruses, without any evidence of associated deleterious effects from existing epidemiological studies and clinical trials. On the other hand, it is known that certain parvoviruses, and especially rodent parvoviruses, are both oncotropic, i.e. accumulate preferentially in neoplastic versus normal tissues, and oncosuppressive, i.e. have a tumor-suppressive effect towards tumor cells, in various animal models. At least part of the oncosuppressive effect is thought to be due to a direct oncolytic action mediated by NS1. This oncosuppressive effect was also demonstrated against human tumor cells transplanted in recipient animals.

It is considered to use parvoviruses for therapeutic purposes. On the one hand, it seems to be of interest to use parvoviruses as vectors for therapeutic genes, i.e. for introducing such genes into the genome of cells. On the other hand, it is considered to use NS1 of parvoviruses as a toxin for treating tumoral diseases. However, initial experiments showed unsatisfactory results.

Therefore, it is the object of the present invention to provide a product by which parvoviruses and NS1 thereof, respectively, can be used for the above purposes.

According to the invention this is achieved by the subject matters defined in the claims.

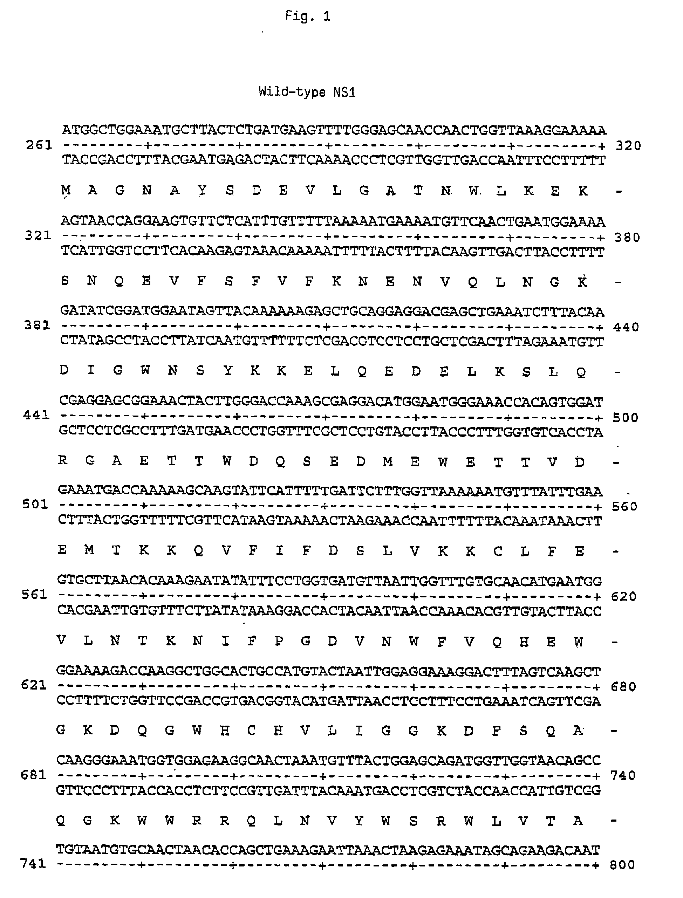

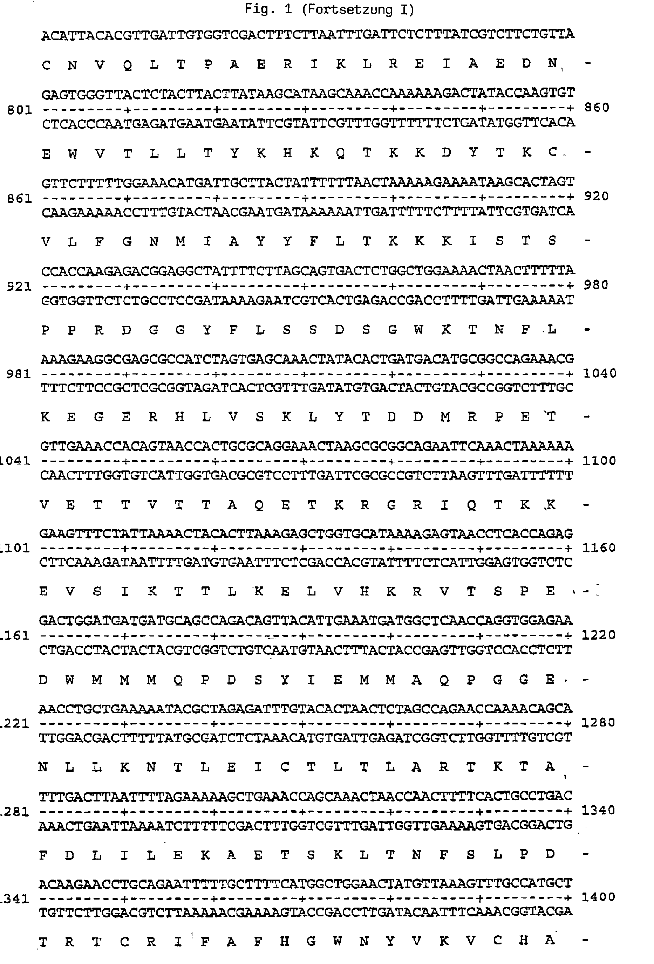

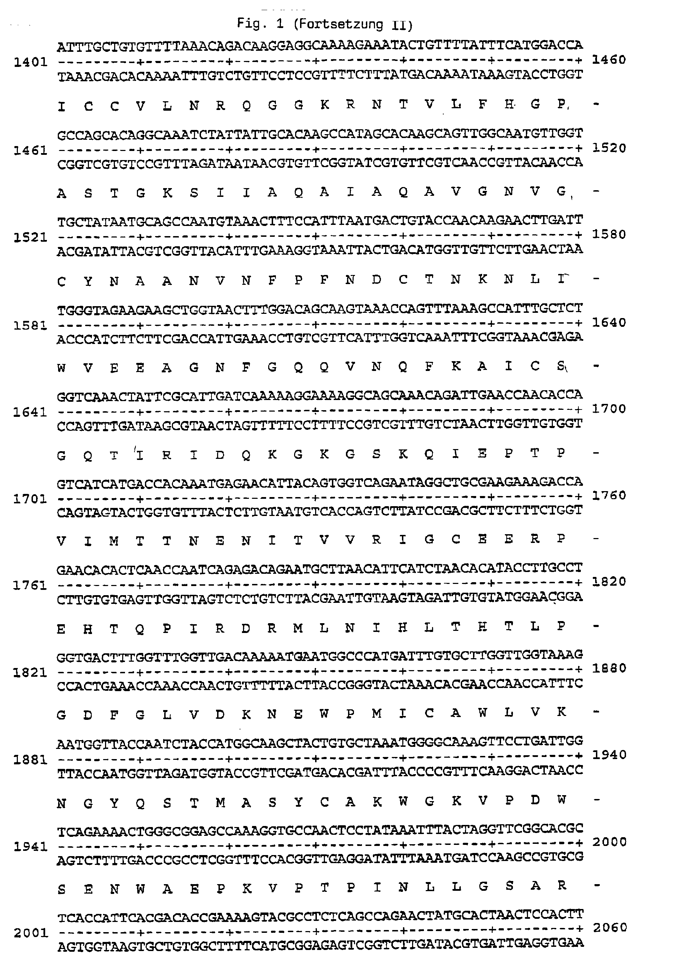

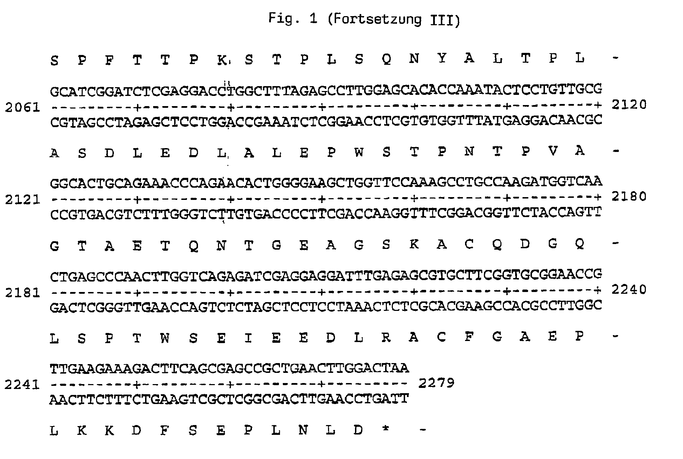

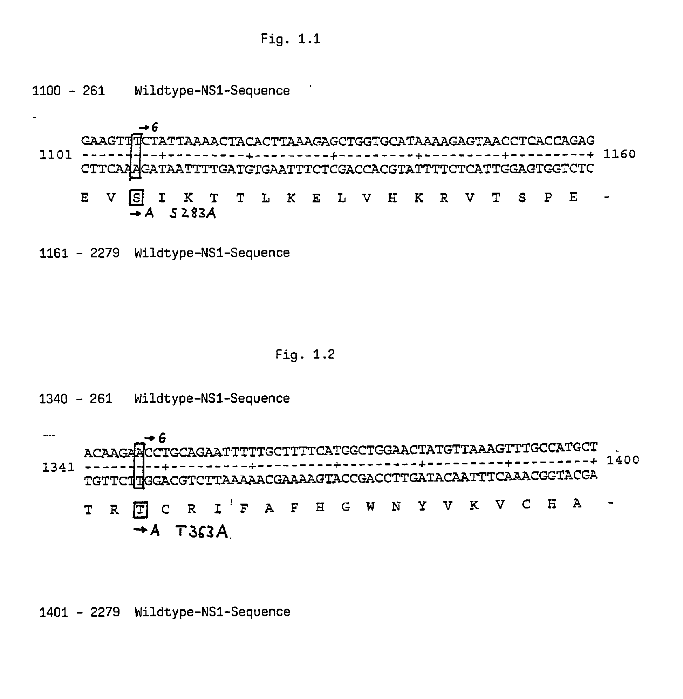

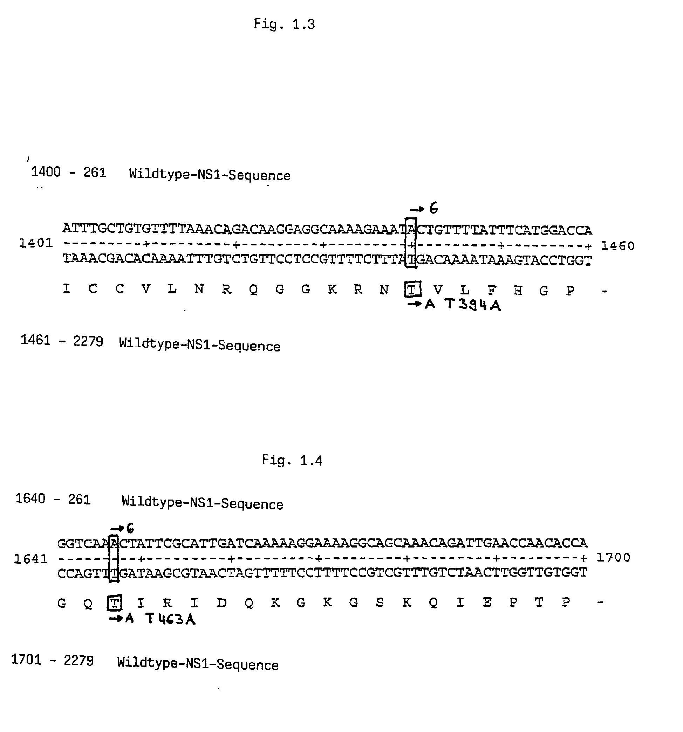

The present invention is based on the applicant's findings that it is possible to interfere with the activities of parvovirus NS1 so as to shift the equilibrium existing between the DNA replication and transcription activities (a) and the cytotoxicity activity (b). In particular, the produced parvovirus NS1 variants in which the DNA replication and transcription activities (a) are reduced and eliminated, respectively, whereas the cytotoxicity activity (b) is maintained or raised. Moreover, the produced parvovirus NS1 variants in which the cytotoxicity activity (b) is reduced and eliminated, respectively, whereas the DNA replication and transcription activities (a) are maintained or raised. Examples of such parvovirus NS1 variants are indicated in Table 1 and figure 1. In addition, the applicant recognized that the above parvovirus NS1 variants and expression vectors coding for them, particularly parvoviruses, respectively, are suitable for therapeutic purposes.

Li et al., Journal of Virology 64 (1990), 4654 - 60 study the effect of a mutation of lysine 405 to serine in the parvovirus H-1 NS1 on DNA replication, late promoter trans activation and cytotoxicity. Nüesch et al., Journal of Virology 72 (1998), 8002 - 12 study the role of NS1 and state that it is unknown which of the phosphorylation sites in NS1 are relevant for activation of the NS1 replicative function. However, both papers do not report on the use of parvovirus NS1 variants having particular mutations at phosphorylation sites for the treatment of tumoral diseases.

According to the invention, the applicant's findings are used to provide a particular use of a parvovirus NS1 variant as defined in the claims.

The expression "parvovirus" comprises any parvovirus, particularly a rodent parvovirus, such as minute virus of mice (MVM) and H-1 virus.

The expression "the equilibrium between the DNA replication and transcription activities (a) and the cytotoxicity activity (b) is shifted" refers to the fact that in a parvovirus NS1 variant according to the invention such an equilibrium is shifted as compared to the parvovirus NS1 wild-type. In particular, the equilibrium can be shifted to the effect that the DNA replication and transcription activities (a) are reduced and eliminated, respectively, whereas the cytotoxicity activity (b) is maintained or raised. The cytotoxicity activity (b) can also be reduced and eliminated, respectively, whereas the DNA replication and transcription activities (a) are maintained or raised. Such an equilibrium can be determined by various methods. As regards the determination of the DNA replication activity, reference is made e.g. to methods described in Legendre and Rommelaere, 1992, J. Virol. 66, 5705; Cotmore et al., 1992, Virology 190, 365; Cotmore et al., 1993, J. Virol. 67, 1579, Cotmore and Tattersall, 1994, Embo. J. 13, 4145. As to the determination of the transcription activity reference is made to methods described e.g. in Rhode and Richards, 1987, J. Virol. 61, 2807. Regarding the determination of the cytotoxicity activity reference is made to the below examples.

According to the invention parvovirus NS1 variants are used in which the shift of equilibrium is achieved by mutation of one or several phosphorylation sites. Particularly preferred are parvovirus NS1 variants which have a mutation at one or several of the phosphorylation sites 283, 363, 394 and 463. Even more preferred are the parvovirus NS1 variants S283A, T363A, T394A and T463A, which are indicated in Table 1 and figure 1. In S283A, a serine is exchanged by an alanine at position 283, in T363A a threonine is exchanged by alanine at position 363, in T394A a threonine is exchanged by alanine at position 394 and in T 463A a threonine is exchanged by alanine at position 463.

The DNA of particular parvovirus NS1 variants was deposited with DSMZ (Deutsche Sammlung von Mikroorganismen and Zellkulturen) on Aug. 11, 1999, i.e. fig. 1.1 as Escherichia coli pRSV-NS:S283A under DSM 12994, fig. 1.2 as Escherichia coli pRSV-NS:T363A under DSM 12995, fig. 1.3 as Escherichia coli pRSV-NS:T394A under DSM 12996 and fig. 1.4 as Escherichia coli pRSV-NS:T463A under DSM 12997.

Such DNA can be present in a vector and expression vector, respectively. A person skilled in the art is familiar with examples thereof. In the case of an expression vector for E. coli these are e.g. pGEMEX, pUC derivatives, pGEX-2T, pET3b, T7 based expression vectors and pQE-8. For the expression in yeast, e.g. pY100 and Ycpadl have to be mentioned while e.g. pKCR, pEFBOS, cDM8, pMSCND, and pCEV4 have to be indicated for the expression in animal cells. The baculovirus expression vector pAcSGHisNT-A is especially suitable for the expression in insect cells.

The vector containing the DNA according to the invention can be a virus, e.g. an adenovirus, vaccinia virus, an AAV virus or a parvovirus, such as MVM or H-1, a parvovirus being preferred. The vector may also be a retrovirus, such as MoMULV, MoMuLV, HaMuSV, MuMTV, RSV or GaLV.

For constructing expression vectors which contain the DNA , it is possible to use general methods known in the art. These methods include e.g. in vitro recombination techniques, synthetic methods and in vivo recombination methods as described in Sambrook et al., supra, for example.

Furthermore, the present invention describes host cells which contain the above described vectors. These host cells include bacteria, yeast, insect and animal cells, preferably mammalian cells. The E. coli strains HB101, DH1, x1776, JM101, JM109, BL21, XLlBlue and SG 13009, the yeast strain Saccharomyces cerevisiae and the animal cells L, A9, 3T3, FM3A, CHO, COS, Vero, HeLa and the insect cells sf9 are preferred. Methods of transforming these host cells, of phenotypically selecting transformants and of expressing the DNA according to the invention by using the above described vectors are known in the art.

### Brief description of the drawings

Fig. 1 shows the DNA and amino acid sequences of parvovirus NS1 variants according to the invention (fig. 1.1, 1.2, 1.3 and 1.4) as compared to a parvovirus NS1 wild-type. In this connection, the mutated sites in the parvovirus NS1 variants according to the invention are labeled each.

The present invention is explained by the examples.

### Example 1: Preparation and purification of NS1 variants according to the invention

The DNA of the NS1 variant S283A according to the invention was provided as an EcoRV to BstEII fragment obtained by chimeric PCR using two mutagenic primers. This fragment was then inserted into the corresponding cleaved expression vector pTHisNS1 (Nuesch et al., Virology 209, (1995), 122) to obtain pTHis NS1:S283A. Such a vector codes for a fusion protein comprising 6 histidine residues (N terminus partner) and S283A of Fig. 1 (C terminus partner). For expression and purification of S283A the NS1 gene under control of the bacteriophage T7 promoter was transferred into vaccinia virus and expressed in eucaryotic cells by double infection together with vTF7-3 (a vaccinia virus expressing the bacteriophage T7 DNA polymerase). 18 hrs post infection cells were harvested and nuclear extracts prepared. The histidine tagged S283A was then purified by affinity chromatography on Ni-NTA agarose and analyzed by 10 % SDS-PAGE (Nuesch et al., supra).

It showed that a parvovirus NS1 variant according to the invention can be prepared in highly pure form.

The NS1 variants T363A, T394A, and T463A were also produced and purified in the same way.

### Example 2: Preparation and detection of an antibody according to the invention

Tubes were coated with purified NS1 variants prepared as in example 1 and monoclonal antibodies (e.g. scFv) specifically binding to S283A were isolated from human synthetic VH+VL scFV phage library (Griffith et al., EMBO J., 13, (1994), 3245) according to standard panning protocols after >5 isolation and amplification procedures. The variable region of the isolated scFv harbored in the phagemid were sequenced to identify NS1 variant interacting partner proteins harboring such binding motifs from comparison with known genes in the gene bank.

It showed that monoclonal antibodies according to the invention can be isolated.

In addition, the NS1 variants were used for immunization of animals in order to obtain poly- or monoclonal antibodies.

### Example 3: Characterization of the parvovirus NS1 variants S283A, T363A, T394A and T463A according to the invention

The characterization of the parvovirus NS1 variants comprised the determination of the DNA replication, transcription, cytotoxicity, DNA binding, nicking and helicase activities. Known methods were used for this purpose (cf. description, supra). As regards the determination of the helicase activity reference is made to Stahl et al. 1986, EMBO J. 5, 1999. As to the determination of the nicking activity reference is made to Christensen et al., 1997, J. Virol. 71, 1405 and Nuesch et al., 1995, supra. Regarding the determination of the DNA binding reference is made to Cotmore et al. 1995, J. Virol. 69, 1652. As far as the determination of the cytotoxicity activity is concerned, the following steps were carried out:
NS1 variants were transferred into an expression vector containing the NS1 gene under the control of the parvovirus MVMP4 promoter (genuine promoter driving the non-structural genes of MVM), and the green fluorescent protein (EGFP) under control of an additional promoter. These constructs were then transfected into A9 cells using lipofectamine (GibcoBRL) according to the manufacturer's instruction and the impact of the NS1 variant on the viability of the cells tested in time course experiments. Transfected cells were identified by fluorescence of the EGFP. Toxic effects were determined in comparison to wild type NS1 or a vector containing no NS1 gene as a function of time as well as a measure of cytopathic changes on the cell morphology.

The data indicated in Table 1 were obtained:

**Table 1**

| | S283A | T363A | T394A | T463A | wt |
|---|---|---|---|---|---|
| P38-TA | + | - | - | ++++ | ++++ |
| ACCA | + | ++++ | ++ | ++ | ++ |
| Nick-1 | + | - | - | +++ | +++ |
| Nick-2 | +++ | - | - | ++++ | ++++ |
| Nick-3 | ++ | - | - | | ++++ |
| Heli | ++ | - | (+) | ++++ | ++++ |
| Rep | + | - | - | + | ++++ |
| Cyto | ++++++ | ++ | +++ | (+) | +++ |

### Example 4: NS1 variants' expression after transduction using recombinant viral vectors

NS1 expression cassettes containing the NS1 variants according to the invention under control of the parvoviral P4 promoter and a 3'untranslated region from parvovirus MVM to ensure stability and translation of the gene product, were transferred either in a parvovirus genome background as exemplified in example 3, or a heterologous viral genome background, such as vaccinia virus (example 1) or adenovirus. Promoter and terminator regions were exchanged according to the requirements. The nucleic acids containing the NS1 variants were then packaged either in vivo (after transient transfection into eucaryotic cells) or in vitro and the packaged transducing particles were isolated. These transducing units containing NS1 variants were used either for studies concerning gene regulation in tissue culture or animals, but also as therapeutic agents either alone or in combination with other agents (such as cytokines) in gene and cancer therapy approaches.

## Claims

1. Use of a parvovirus NS1 variant having a shifted equilibrium between the DNA replication and transcription activities (a) and the cytotoxicity activity (b), wherein one or several phosphorylation sites are mutated by amino acid substitution causing the shift of equilibrium; and
(i) the activities (a) are reduced or eliminated, and activity (b) is maintained or increased or
(ii) the activities (a) are maintained and activity (b) is reduced or eliminated for the preparation of a pharmaceutical composition for treating tumoral diseases.

2. The use of claim 1, wherein the parvovirus NS1 variant is expressed by an expression vector comprising a DNA coding for the parvovirus NS1 variant.

3. The use of claim 2, wherein the expression vector is parvovirus H1.

4. The use according to claim 1 wherein the mutations are located at sites 283, 363, 394 and/or 463.

5. The use according to claim 4, namely the NS1 variants S283A, T363A, T394A and T463A.

## Patentansprüche

1. Verwendung einer Parvovirus NS1 Variante mit einem verschobenen Gleichgewicht zwischen den DNA-Replikations- und Transkriptionsaktivitäten (a) und der Zytotoxizitätsaktivität (b), wobei eine oder mehrere Phosphorylierungsstellen durch eine Aminosäuresubstitution mutiert sind, was die Verschiebung des Gleichgewichts verursacht, und
(i) die Aktivitäten (a) reduziert oder ausgeschaltet sind und die Aktivität (b) aufrechterhalten oder erhöht ist oder
(ii) die Aktivitäten (a) aufrechterhalten sind und die Aktivität (b) reduziert oder ausgeschaltet ist,
zur Herstellung einer pharmazeutischen Zusammensetzung zur Behandlung von Tumorerkrankungen.

2. Verwendung nach Anspruch 1, wobei die Parvovirus NS1 Variante durch einen Expressionsvektor exprimiert wird, der eine für die Parvovirus NS1 Variante kodierende DNA umfasst.

3. Verwendung nach Anspruch 2, wobei der Expressionsvektor das Parvovirus H1 ist.

4. Verwendung nach Anspruch 1, wobei sich die Mutationen an den Stellen 283, 363, 394 und/oder 463 befinden.

5. Verwendung nach Anspruch 4, nämlich der NS1 Varianten S283A, T363A, T394A und T463A.

## Revendications

1. Utilisation d'un variant du Parvovirus NS1 ayant un équilibre décalé entre les activités de réplication et de transcription d'ADN (a) et l'activité de cytotoxicité (b), pour laquelle un ou plusieurs sites de phosphorylation font l'objet d'une mutation par un remplacement d'acide aminé provoquant le décalage d'équilibre ; et
i) les activités (a) sont réduites ou éliminées et l'activité (b) est maintenue ou accrue ou
ii) les activités (a) sont maintenues et l'activité (b) est réduite ou éliminée pour la préparation d'une composition pharmaceutique pour le traitement de maladies tumorales.

2. Utilisation selon la revendication 1, pour laquelle le variant du Parvovirus NS1 est exprimé par un vecteur d'expression comprenant un codage d'ADN pour le variant du Parvovirus NS 1.

3. Utilisation selon la revendication 2, pour laquelle le vecteur d'expression est un Parvovirus H1.

4. Utilisation selon la revendication 1, pour laquelle les mutations sont localisées aux sites 283, 363, 394 et/ou 463.

5. Utilisation selon la revendication 4, à savoir des variants NS1 S283A, T363A, T394A et T463A.
